# EUROPEAN PATENT APPLICATION

(11) **EP 2 422 716 A1**
(43) Date of publication of application: **29.02.2012**
(21) Application number: 10767060.6
(22) Date of filing: 20.04.2010
(51) Int. Cl.: A61B 17/12

(54) **EMBOLIZATION COIL**

(30) Priority: 20.04.2009 JP 2009102501
(71) Applicant: Kaneka Corporation, Osaka-shi, Osaka 530-8288 (JP)
(72) Inventor: YAMANAKA, Yasushi, Ashigarakami-gun Kanagawa 258-0113 (JP); NOICHI, Naoko, Ashigarakami-gun Kanagawa 258-0113 (JP); OGAWA, Atsushi, Yamakita-machi Ashigarakami-gun Kanagawa 2580113 (JP)
(74) Representative: TBK
(86) International application number: PCT/JP2010/057010
(87) International publication number: WO 2010/123003

(57) **Abstract**

Provided is an embolization coil in which even if the coil is wound in a shape of a small diameter, an elongation-preventing wire is not broken and the delivery property thereof can be prevented from deterioration. An embolization coil has a coil of partial or full-pitch winding and an elongation-preventing wire which are fixed respectively at two points different from each other, and is **characterized in that** the natural length of the coil arranged between two different points before the coil is fixed is longer than the length of the elongation-preventing wire arranged between two different points before the elongation-preventing wire is fixed.

## Description

### Technical Field

The present invention relates to an embolization coil for use in intravascular treatment. More specifically, it relates to an embolization coil for use in treatment of cerebral aneurysm or embolism of blood vessel.

### Background Art

Currently, intravascular treatments such as those using a catheter have been known as less-invasive treatment methods for intravascular lesions such as aneurysm. The embolization coil used intravascularly in these intravascular treatment methods is needed to show various characteristics such as those shown below.

First, the embolization coil should be flexible enough and show required placement efficiency in placement operation to prevent problems such as a damage of the wall of blood vessel or aneurysm caused by excessively large load applied to the site of blood vessel or aneurysm.

The embolization coil should also have a function to prevent unlimited elongation of itself, which function is required for reliable operation for repositioning or relocation of the embolization coil recovered from the lesion site after its placement.

Various developments had been made to obtain these characteristics. Examples of the developments include improvement of the placement efficiency of the embolization coil allowing rapid bending thereof when connected to the vascular wall, which is made possible by making the terminal of the embolization coil more flexible than the middle region, as disclosed in Patent Document 1, and provision of a function to prevent unlimited elongation of the embolization coil itself, which is made possible by installing an elongation-preventing member in the embolization coil, as disclosed in Patent Document 2.

However in the case of the configuration of Patent Document 1, it is not possible to prevent unlimited elongation of the embolization coil main body because there is no elongation-preventing member and the delivery efficiency of the embolization coil (convenience of operation in relocation of the embolization coil in catheter, relocation and repositioning after recovery, and others) may be reduced by a terminal pitch-wound region. Also, in the case of the configuration of Patent Document 2 (where the total length of the elongation-preventing member is longer than the coil length of the embolization coil main body in a normal state), there were still many problems to overcome for prevention of rupture of the elongation-preventing member (e.g., prevention of rupture when the coil is wound into a shape with a smaller diameter).

### Citation List

### Patent Literature

Patent Document 1: JP-A No. 08-336600
Patent Document 2: JP-A No. 2004-215797

### Summary of Invention

### Technical Problem

An object of the present invention is to provide an embolization coil that has an elongation-preventing wire which will not be broken nor deteriorated in delivery efficiency even if the coil is wound into a shape with a small diameter.

### Solution to Problem

Provided is an embolization coil including a partially or entirely pitch-wound coil and an elongation-preventing wire that are fixed to each other at two different points, wherein the natural length of the coil placed between the two different points before fixation is longer than the length of the elongation-preventing wire placed between the two different points before fixation. It is possible by pitch-winding a part or all of the coil to prevent rupture of the elongation-preventing wire (particularly when the embolization coil is wound into a shape with a smaller diameter), and to reduce deterioration in delivery efficiency caused by pitch winding by bringing the natural length of the coil and the length of the elongation-preventing wire into a particular relationship.
In the present invention, the "two different points," as used in the present invention, means two points separated in the axial direction when the coil is linear.

Also provided is the embolization coil, wherein the natural length of the coil placed between the two different points before fixation is more than 100% and not more than 130% with respect to 100% of the length of the elongation-preventing wire placed between the two different points before fixation. It is possible to prevent rupture of the elongation-preventing wire by repeated bending and deterioration in delivery efficiency caused by pitch winding, by bringing the natural length of the coil before fixation and the length of the elongation-preventing wire before fixation into a particular relationship.

Also provided is the embolization coil, wherein the pitch distance in the pitch-wound region of the partially or entirely pitch-wound coil is 20% or more of the external diameter of the wire forming the coil. It is possible to relax the excessive elongation force applied to the elongation-preventing wire and thus prevent rupture of the elongation-preventing wire by forming a pitch-wound region with a particular distance.
The "pitch distance," as used in the present invention, means the distance between neighboring wires in the pitch-wound coil.

Also provided is the embolization coil, wherein the partially pitch-wound coil is a coil in which the length of the pitch-wound region is 5-40% of the natural length of the coil before fixation.

Also provided is the embolization coil, wherein the pitch-wound region of the partially or entirely pitch-wound coil is located at a proximal side of the coil.

Also provided is the embolization coil, wherein the partially or entirely pitch-wound coil is further wound helically, forming a secondary coil.

Also provided is the embolization coil, wherein the partially or entirely pitch-wound coil has additionally a three-dimensional shape.

Also provided is the embolization coil, wherein the partially or entirely pitch-wound coil is made of a metal coil in which a metal wire is wound helically.

Also provided is the embolization coil, wherein the metal wire is made of platinum or a platinum alloy.

Also provided is the embolization coil, wherein the elongation-preventing wire is made of a metal wire.

Also provided is the embolization coil, wherein the metal wire is made of platinum or a platinum alloy.

### Advantageous Effects of Invention

The present invention provides an embolization coil having an elongation-preventing wire not broken even when the embolization coil is wound into a shape with a smaller diameter. The present invention also provides an embolization coil resistant to deterioration in delivery efficiency.

### Brief Description of the Drawings

Figure 1 is a schematic cross-sectional view illustrating a component, coil 1, in an embodiment of the embolization coil according to the present invention, and a schematic diagram illustrating another component, an elongation-preventing wire 2, in the embodiment of the embolization coil according to the present invention.
Figure 2 is a schematic cross-sectional view illustrating an example of the embolization coil 20 according to the present invention that is prepared by fixing the coil 1 and the elongation-preventing wire 2 shown in Figure 1 to each other.
Figure 3 is a cross-sectional view illustrating an example of a method of connecting the embolization coil 21 according to the present invention to a wire 9.
Figure 4 is a schematic plan view illustrating an example of the embolization coil 22 according to the present invention having a secondary coil formed by helical winding.

### Description of Embodiments

The present invention relates to an embolization coil including a partially or entirely pitch-wound coil and an elongation-preventing wire that are fixed to each other at two different points, wherein the natural length of the coil placed between the two different points before fixation is longer than the length of the elongation-preventing wire placed between the two different points before fixation.

The embolization coil in the configuration above may be defined as an embolization coil having a partially or entirely pitch-wound coil and an elongation-preventing wire that are fixed to each other at two different points, wherein the natural length of the coil placed between the two different points before fixation is longer than the length between the different two points.

Alternatively in the embolization coil in the configuration above, the coil and the elongation-preventing wire are connected to each other so that the pitch-wound region of the partially or entirely pitch-wound coil is compressed in an axial direction by the elongation-preventing wire.

The coil for use in the present invention is partially or entirely pitch-wound, thus preventing rupture of the elongation-preventing wire (particularly when the embolization coil is wound into a shape with a smaller diameter). For example if an embolization coil in a secondary coil shape having a diameter extremely larger in common-sense terms than the diameter of the aneurysm is placed, there is a possibility of an elongation-preventing wire being ruptured because of its insufficient length, but in the structure, the insufficiency of the elongation-preventing wire is compensated by shrinkage of the pitch-wound region, preventing such rupture.

The pitch-wound region in the partially or entirely pitch-wound coil may be formed in the step of coiling, but alternatively, the pitch-wound region may be formed by first preparing a densely wound coil and then elongating any region thereof in the axial direction. A pitch distance of the pitch-wound region of 20% or more with respect to the external diameter of the wire forming the coil is effective in preventing rupture of the elongation-preventing wire more reliably. Since the delivery efficiency of the coil often declines as the pitch distance increases, the pitch distance is preferably not larger than 25% of the external diameter of the wire.
As described above, the "pitch distance," as used in the present invention, means the distance between neighboring wires in pitch-wound coil, and the distance between wires may be uniform entirely over the pitch-wound region or may vary as needed.

The coil for use in the present invention may be pitch-wound entirely but, if the length of the coil in the axial direction is natural length when the coil is placed for example in a linear groove without any force applied in the axial direction, it is possible to reduce the load applied to the elongation-preventing wire and thus to prevent rupture of the elongation-preventing wire effectively by pitch-winding the region corresponding to 5 to 40% of the natural length of the coil. If the coil is pitch-wound at a rate of 5 to 40% with respect to the natural length, the pitch-wound region may be distributed to multiple positions of the coil, but it is preferable to pitch-wind a region of the proximal side of coil (the proximal side being the side of the coil closer to the surgeon during treatment) where the elongation-preventing wire is insufficient, particularly when an embolization coil having a large secondary shape is wound into a shape with a smaller diameter.

The coil for use in the present invention preferably has a secondary coil shape, in which the partially or entirely pitch-wound coil is wound further helically or three-dimensionally, thus occupies a certain or more space without aggregation in blood vessel when placed therein.
The secondary coil formed by "helical winding" means various shapes formed by the pitch-wound coil, as it is wound helically, and examples thereof include three-dimensional shapes such as mostly cylindrical, mostly conical, mostly spherical and mostly array-like, and also the shape in which the coil is wound spirally on the same plane.
The three-dimensional shape is not particularly limited, and examples thereof include the shape in which the partially or entirely pitch-wound coil is wound helically and also the shapes in which the coil is wound in a modified direction or as it is bent irregularly, such as spherical, elliptical spherical, conical, cubic, rectangular and various other shapes.

The coil for use in the present invention can be prepared from a biocompatible raw material such as metal wire or resin, and in particular metal wires, which mainain its stable three-dimensional-shape, are preferable (in particular, metal coils of helically wound metal wire). Examples of the biocompatible metal wire materials include platinum, tungsten, titanium, gold, the alloys thereof, stainless steel and the like. In particular, the metal wire material is preferably platinum or a platinum alloy.

In the present invention, the natural length of the coil placed between the two different points before fixation is preferably adjusted to be more than 100% and not larger than 130%, with respect to 100% of the length of the elongation-preventing wire placed between the two different points before fixation. It is possible, by making the length of the elongation-preventing wire not longer than the natural length of the coil in this way, to prevent the rupture of the elongation-preventing wire caused by repeated bending applied to the elongation-preventing wire by change in position and replacement during placement of the embolization coil. Also by making the length of the elongation-preventing wire shorter than the natural length of the coil and thus making the coil pitch-wound region shrunk, which leads to reduction of the flexibility of the coil and the friction with the catheter internal wall, it is possible to reduce the deterioration in delivery efficiency caused by pitch winding. In particular, it is more preferable to make the natural length of the coil placed between the two different points before fixation larger than 100% and not larger than 105% of the length of the elongation-preventing wire placed between the two different points before fixation. The elongation-preventing wire may not be used alone, and a single elongation-preventing wire may be wound reciprocally multiple times between two points, before fixing it to the coil.

As described above, the "two different points," as used in the present invention, means two points separated in the axial direction when the coil is linear. The positions of the two points are not particularly limited, if the advantageous effects of the present invention are obtained. Examples of the positions of the two points include both terminals of the coil, any two points in the axial direction between both terminals of the coil, one terminal of the coil and any position separated therefrom between the terminals of the coil in the axial direction, and the like.

The elongation-preventing wire for use in the present invention can be prepared from a biocompatible raw material such as metal wire or resin and, in particular, metal wires, which are resistant to elongation force applied in the axial direction, are preferable. Examples of the materials for the biocompatible metal wire (metal wire) include platinum, tungsten, titanium, gold, the alloys thereof, stainless steel and the like. In particular, the metal wire is preferably made of platinum or a platinum alloy.

Various methods are possible as the methods of placing the elongation-preventing wire in the coil for use in the present invention, but it is preferable to place and fix the elongation-preventing wire in the region from one terminal of the coil to the other, in order to make the entire coil resistant to elongation. For example, one terminal of the elongation-preventing wire is fixed directly or indirectly to one terminal of the coil, and the other terminal of the elongation-preventing wire directly or indirectly to the other terminal of the coil.
The location of the elongation-preventing wire, when the elongation-preventing wire is fixed to the coil, is not particularly limited if the advantageous effects of the present invention are obtained, and in an embodiment, it is placed in the lumen region of the coil.

The method of fixing the elongation-preventing wire to the coil is not particularly limited, and adhesion with an adhesive, fusion, pressurization, physical fixation and other direct or indirect means can be used. For example, by inserting one terminal of the wire constituting the elongation-preventing wire into a ring having an internal diameter sufficiently larger than the diameter of the wire constituting the elongation-preventing wire, bending the wire, inserting the wire into the lumen region of the coil, connecting the ring to one terminal of the coil, and fixing both terminals of the wire to the other terminal of the coil by a known direct or indirect means, it is possible to fix the elongation-preventing wire indirectly to the coil at high degree of freedom without directly fixing it to at least one terminal of the coil (ring-connected terminal) and to reduce the elongation or compressive force applied to the elongation-preventing wire during placement of the embolization coil. One terminal of the two different points is then the region where the wire is inserted into the ring, and the other terminal is the region in which the both terminals of the wire are fixed.

The embolization coil according to the present invention is preferably connected to a delivery jig such as delivery wire or catheter, delivered to the placement site in blood vessel as connected to the delivery jig, and placed there in a desired shape. The delivery jig and the embolization coil are connected to each other, for example, by a method of mechanically connecting the embolization coil to the delivery jig, for example by screwing, so that the embolization coil can be mechanically separated after its placement in blood vessel. Alternatively, they can be connected to each other, for example, by a method of connecting the embolization coil to the delivery jig by connection with a connecting material that is cleavable under heat so that the connecting material can be cleaved or separated by the heat generated by supply of electric current after its placement in blood vessel, or by a method of connecting the embolization coil to the delivery jig by connection with an electrolyzable connecting material so that the connecting material is separated by electrolysis, as electric current is supplied, after placement of the embolization coil in blood vessel.

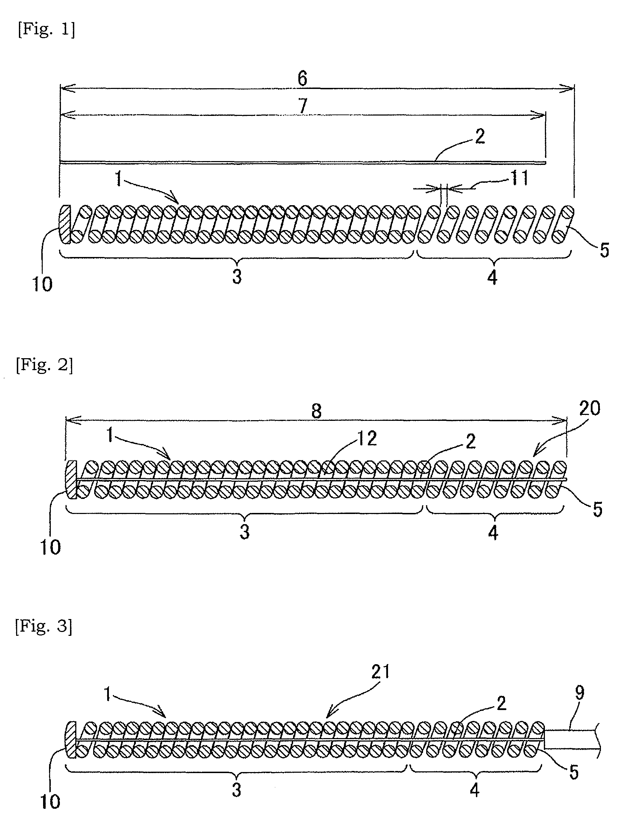

Hereinafter, the present invention will be described with reference to drawings. Figure 1 is a schematic view illustrating the components in an embodiment of the embolization coil according to the present invention before fixation. Specifically, it is a crosssectional view illustrating a coil 1 and a schematic view illustrating an elongation-preventing wire 2. The coil 1 shown in Figure 1 has a densely wound region 3 partially and a pitch-wound region 4 at the proximal side 5 of the coil 1. The natural length 6 of coil before fixation is longer than the length 7 of the elongation-preventing wire before fixation. A head unit 10 is connected to one terminal of the coil 1. The pitch distance (distance between wires) is, for example, the distance indicated by numerical reference 11 in Figure 1.

Figure 2 is a crosssectional view schematically illustrating the components in an embodiment of the embolization coil 20 according to the present invention after fixation, wherein the elongation-preventing wire 2 is placed in the lumen region 12 of the coil 1 and the coil 1 and the elongation-preventing wire 2, which are different in length (respectively, 6 and 7) before fixation, are fixed at the same length 8. Terminals of the coil 1 and the elongation-preventing wire 2 are fixed indirectly by a head unit 10, and the other terminals of the coil 1 and the elongation-preventing wire 2 are fixed directly or indirectly to each other, although the configuration is not shown in the Figure.

Figure 3 is a crosssectional view illustrating an example of the method of connecting the embolization coil according to the present invention 21 to a delivery wire 9. The distal terminals of the coil 1 and the elongation-preventing wire 2 are fixed to each other via the head unit 10, and the proximal terminals of the coil 1 and the elongation-preventing wire 2 are fixed to each other via the wire 9.

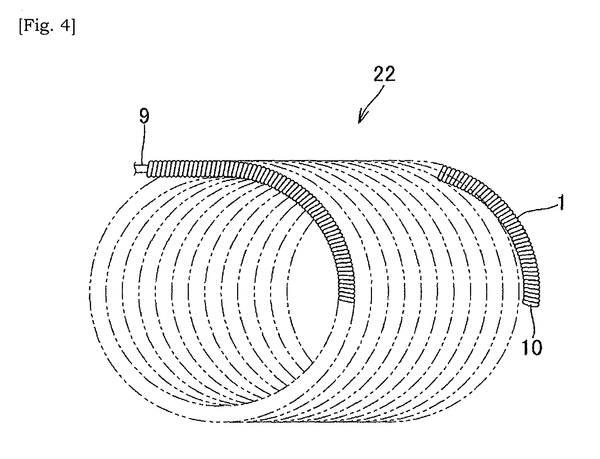

Figure 4 is a schematic top view illustrating an example of the embolization coil according to the present invention in the shape of secondary coil, in which the coil is wound helically. In the embolization coil 22 shown in the present embodiment, the coil 1, as shown in Figure 3, having a head unit 10 connected at the distal terminal, a wire 9 connected to the coil proximal side, and an elongation-preventing wire placed in the coil lumen region (not shown in Figure) is wound mostly cylindrically, densely and helically. The pitch-wound region of the coil 1 is not shown in the Figure 4.

### Examples

Hereinafter, Examples and Comparative Examples, which are performed for confirmation of the operational advantages of the embolization coil according to the present invention, will be described.

### (Example 1)

A metal coil (coil diameter: 0.25 mm) of a platinum-tungsten alloy wire (external diameter: 0.04 mm), which is a pitch-wound coil having a natural length of 200 mm, having a pitch-wound region with a pitch distance of 30% of the external diameter of the platinum-tungsten alloy wire in the region from one terminal to 30-mm inside of the coil and a densely wound region in the other region, and an elongation-preventing wire of a platinum-tungsten alloy wire (external diameter: 0.01 mm) were fixed to each other between different two points, to give an embolization coil in a helical secondary shape (mostly cylindrical) having a diameter of 16.0 mm. The natural length of the coil placed between the two different points before fixation was adjusted to be 102% of the length of the elongation-preventing wire placed between the two different points before fixation. The two different points mean both terminals of the metal coil.

### (Comparative Example 1)

Similarly to Example 1, a metal coil (coil diameter: 0.25 mm) of a platinum-tungsten alloy wire (external diameter 0.04 mm), which is a pitch-wound coil having a natural length of 200 mm, having a pitch-wound region with a pitch distance of 30% of the external diameter of the platinum-tungsten alloy wire in the region from the terminal to 30-mm inside of the coil and a densely wound region in the other region, and an elongation-preventing wire of a platinum-tungsten alloy wire (external diameter: 0.01 mm) were fixed to each other between two different points, to give an embolization coil in a helical secondary shape (mostly cylindrical) having a diameter of 16.0 mm. The natural length of the coil placed between the two different points before fixation was then adjusted to be the same as the length of the elongation-preventing wire placed between the two different points before fixation. The two different points mean both terminals of the metal coil.

### (Comparative Example 2)

A densely wound metal coil (coil diameter: 0.25 mm, natural length: 200 mm) of a platinum-tungsten alloy wire (external diameter: 0.04 mm) and an elongation-preventing wire of a platinum-tungsten alloy wire (external diameter: 0.01 mm) were fixed to each other between two different points to give an embolization coil in a helical secondary shape (mostly cylindrical) having a diameter of 16.0 mm. The natural length of the coil placed between the two different points before fixation was then adjusted to be the same as the length of the elongation-preventing wire placed between the two different points before fixation. The different two points mean both terminals of the metal coil.

Each of the samples obtained in Example 1 and Comparative Examples 1 and 2 were placed in an aneurysm silicone model (aneurysm internal diameter: 4 mm) and then withdrawn from it, and then, rupture of the elongation-preventing wires was examined. In addition, each of the samples obtained in Example 1 and Comparative Examples 1 and 2 were pushed and pulled five times in a catheter; the force applied to the hand then was measured with a force gauge; and the average was used as the indicator of lubricity. The lower the lubricity indicator value, the better the delivery efficiency. The results are summarized in Table 1.
The phrase "[samples were] pushed and pulled in a catheter" specifically means the followings: A sample is placed in a common microcatheter in such a manner that the distal terminal of the sample is located at a position approximately 10 cm backward from the distal opening of the catheter; the sample is pushed forward from the position described above until the region of approximately 10 cm from the sample distal region protrudes outside the distal opening of the microcatheter (as the proximal-sided region of the sample remains in the catheter); and the sample is pulled backward until the distal terminal of the sample is located at a position approximately 10 cm proximal from the distal opening of the microcatheter in the catheter. The series of operations, as a unit of reciprocation, was repeated five times.
The "average" thereof means the average of five maximum values obtained in repeated reciprocal operations.

**[Table 1]**

| | Example 1 | Comparative Example 1 | Comparative Example 2 |
|---|---|---|---|
| Elongation-preventing wire after placement in aneurysm model | Not broken | Not broken | Broken |
| Lubricity indicator (N) | 0.42 | 0.67 | 0.32 |

The results shown in Table 1 confirmed that it is possible to prevent the rupture of the elongation-preventing wire in the embolization coil according to the present invention (Example 1) that may occur, for example, when an embolization coil in a secondary coil shape unimaginably larger in current common-sense terms with respect to the diameter of the aneurysm is placed. The results also confirmed that it is possible to prevent deterioration in delivery efficiency, as indicated by the lubricity indicator. The results also confirmed that, although it is possible to prevent the rupture of elongation-preventing wire by forming a pitch-wound region in the coil, as shown in Comparative Examples 1 and 2, the delivery efficiency declines, as indicated by the lubricity indicator. It was also shown that the elongation-preventing wire is broken when a coil has no pitch-wound region. In contrast, in the case of the embolization coil according to the present invention (Example 1), it is possible, differently from Comparative Example 1, to reduce the deterioration in delivery efficiency caused by pitch winding, because the pitch-wound region of the coil is compressed by the elongation-preventing wire in the axial direction, reducing flexibility of the coil.

### Reference Signs List

- 1: Coil
- 2: Elongation-preventing wire
- 3: Densely wound region
- 4: Pitch-wound region
- 5: Coil proximal side
- 6: Natural length of coil before fixation
- 7: Length of elongation-preventing wire before fixation
- 8: Length of embolization coil after fixation
- 9: Delivery wire
- 10: Head unit
- 11: Pitch distance (distance between wires)
- 12: Lumen region
- 20, 21, 22: Embolization coil

## Claims

1. An embolization coil comprising a partially or entirely pitch-wound coil and an elongation-preventing wire, the coil and the elongation preventing wire fixed to each other at two different points, wherein the natural length of the coil placed between the two different points before fixation is longer than the length of the elongation-preventing wire placed between the two different points before fixation.

2. The embolization coil according to Claim 1, wherein the natural length of the coil placed between the two different points before fixation is greater than 100% and not more than 130% with respect to 100% of the length of the elongation-preventing wire placed between the different two points before fixation.

3. The embolization coil according to Claim 1 or 2, wherein the pitch distance in the pitch-wound region of the partially or entirely pitch-wound coil is 20% or more of the external diameter of the wire forming the coil.

4. The embolization coil according to any one of Claims 1 to 3, wherein the partially pitch-wound coil is a coil in which the length of the pitch-wound region is 5 to 40% of the natural length of the coil before fixation.

5. The embolization coil according to any one of Claims 1 to 4, wherein the pitch-wound region of the partially or entirely pitch-wound coil is located at a proximal side of the coil.

6. The embolization coil according to any one of Claims 1 to 5, wherein the partially or entirely pitch-wound coil is further wound helically, forming a secondary coil.

7. The embolization coil according to any one of Claims 1 to 5, wherein the partially or entirely pitch-wound coil has additionally a three-dimensional shape.

8. The embolization coil according to any one of Claims 1 to 7, wherein the partially or entirely pitch-wound coil is a metal coil of a metal wire wound helically.

9. The embolization coil according to Claim 8, wherein the metal wire is made of platinum or a platinum alloy.

10. The embolization coil according to any one of Claims 1 to 9, wherein the elongation-preventing wire is made of a metal wire.

11. The embolization coil according to Claim 10, wherein the metal wire is made of platinum or a platinum alloy.
